# EUROPÄISCHE PATENTSCHRIFT

(11) **EP 0 732 081 B1**
(45) Veröffentlichungstag und Bekanntmachung des Hinweises auf die Patenterteilung: **03.05.2000**
(21) Anmeldenummer: 96102698.6
(22) Anmeldetag: 22.02.1996
(51) Int. Cl.: A61B 17/70

(54) **Verankerungselement**
Anchor element
Elément d'ancrage

(30) Priorität: 15.03.1995 DE 19509332
(43) Veröffentlichungstag der Anmeldung: 18.09.1996
(73) Patentinhaber: Biedermann, Lutz, 78048 Villingen-Schwenningen (DE); Harms, Jürgen, Prof. Dr., 76337 Waldbronn (DE)
(72) Erfinder: Biedermann, Lutz, 78048 Villingen-Schwenningen (DE); Harms, Jürgen, Prof. Dr., 76337 Waldbronn (DE)
(74) Vertreter: Prüfer, Lutz H., Dipl.-Phys.

(56) Entgegenhaltungen:
- EP-A- 0 382 256
- WO-A-91/01115
- DE-C- 4 307 576

## Beschreibung

Die Erfindung betrifft ein Verankerungselement nach dem Oberbegriff des Patentanspruches 1.

Ein derartiges Verankerungselement in Form einer Polyaxial-Knochenschraube ist aus der DE 43 07 576 C1 bekannt. Bei dieser Vorrichtung wird die Stellung des Schraubenelementes relativ zu dem Aufnahmeteil durch eine auf den Kopf des Schraubenelementes einwirkende Druckscheibe, welche durch einen eingesetzten Korrekturstab und eine Stabfixierschraube gegen den Kopf des Schraubenelementes gedrückt wird, fixiert. Dabei tritt das Problem auf, daß beim Lösen der Stabfixierschraube zum Positionieren von Stab und Verankerungselement relativ zueinander gleichzeitig die Blockierung des Kopfes des Schraubenelementes gegenüber dem Aufnahmeteil aufgehoben wird. Somit wird jedesmal bei einem Verstellen der Lage des Aufnahmeteiles auf dem die Wirbel verbindenden Stab auch das Aufnahmeteil gegenüber dem Kopf des Schraubenelementes verstellt, was kontraproduktiv ist.

Aufgabe der Erfindung ist es, ein Verankerungselement zu schaffen, bei dem beim Justieren der Stellung des Aufnahmeteiles relativ zu dem Stab die Blockierung zwischen dem Kopf des Schraubenelementes und dem Aufnahmeteil erhalten bleibt.

Die Aufgabe wird durch das in Patentanspruch 1 gekennzeichnete Verankerungselement gelöst.

Weiterbildungen der Erfindung sind in den Unteransprüchen gegeben.

Weitere Merkmale und Zweckmäßigkeiten der Erfindung ergeben sich aus der Beschreibung von Ausführungsbeispielen anhand der Figuren.

Von den Figuren zeigen:
- Fig. 1: eine Querschnittsansicht des Verankerungselementes mit einzulegendem Stab, teilweise in geschnittener Darstellung;
- Fig. 2: eine Detailansicht des Verankerungselementes in sionsdarstellung; und
- Fig. 3: eine zum Teil geschnittene Darstellung des kerungselementes mit eingesetztem Schraubenelement und Druckelement.

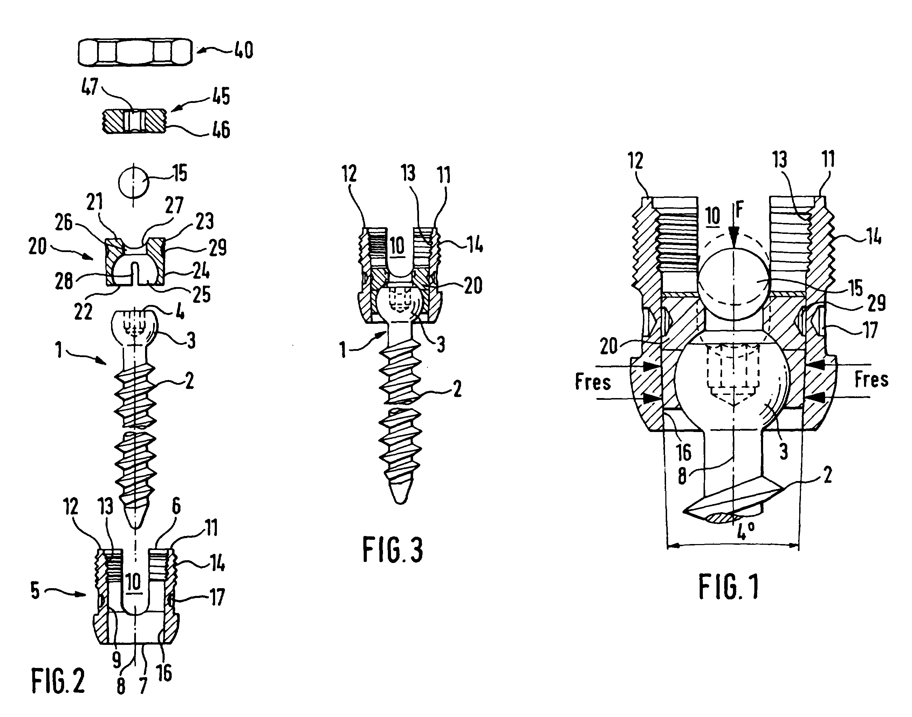

Wie am besten aus den Figuren 2 und 3 ersichtlich ist, weist ein Verankerungselement das eigentliche Schraubenelement 1 mit einem Gewindeschaft 2 und einem Kopf 3 auf. Der Kopf 3 ist angrenzend an den Gewindeschaft 2 kugelsegmentförmig ausgebildet. In axialer Richtung ist die Länge des Kopfes 3 größer als der Radius des Kopfes. Koaxial zur Gewindeachse und auf dem dem Gewindeschaft 2 gegenüberliegenden Ende weist der Kopf 3 auf seiner Stirnseite eine Ausnehmung 4 zum Ineingriffbringen mit einem Imbusschlüssel auf.

Das Verankerungselement umfaßt ferner ein Aufnahmeteil 5 für die Aufnahme des Kopfes 3 des Schraubenelementes 1. Das Aufnahmeteil 5 weist ein erstes Ende 6 und ein diesem gegenüberliegendes zweites Ende 7 und eine das erste und das zweite Ende schneidende Symmetrieachse 8 auf. Es ist eine zu der Symmetrieachse koaxiale Bohrung 9 zum Hindurchführen des Gewindeabschnittes 2 von dem ersten Ende her vorgesehen. In einem an das erste Ende 6 angrenzenden ersten Bereich hat das Aufnahmeteil 5 eine zur Symmetrieachse 8 symmetrisch angeordnete U-förmige Ausnehmung 10, deren Grund zu dem zweiten Ende 7 hin gerichtet ist und deren beide freie Seitenschenkel 11, 12 sich zu dem ersten Ende 6 hin erstrecken. In dem Abschnitt der Schenkel 11, 12 ist die Bohrung zylindrisch, und das Aufnahmeteil 5 weist in diesem Abschnitt jeweils ein Innengewinde 13 und ein Außengewinde 14 auf. Der durch die U-förmige Ausnehmung 10 gebildete Kanal ist gerade so groß, daß ein mehrere solche Verankerungselemente verbindender Stab 15 eingelegt werden kann. In einem an das zweite Ende 7 angrenzenden zweiten Bereich schließt sich an den zylindrischen Abschnitt der Bohrung 9 ein Abschnitt 16 an, in dem die Bohrung 9 sich mit einem Kegelwinkel von etwa 4° gegen das zweite Ende 7 hin konisch verjüngt. Die axiale Länge dieses Abschnittes 16 entspricht in etwa der Abmessung des Kopfes 3 in axialer Richtung.

Der Durchmesser der Bohrung 9 ist an dem zweiten Ende wesentlich größer als der des Gewindeschaftes 2, so daß letzterer im eingesetzten Zustand innerhalb eines Kegelwinkels um die Symmetrieachse 8 schwenkbar ist.

Zum Fixieren der Stellung des Kopfes 3 in dem Aufnahmeteil 5 ist ein auf den Kopf 3 des Schraubenelementes einwirkendes Druckelement 20 mit einem dem Kopf 3 abgewandten ersten Ende 21 und einem diesem gegenüberliegenden zweiten Ende 22 vorgesehen. Das Druckelement weist angrenzend an das erste Ende 21 einen im wesentlichen zylinderförmig ausgebildeten ersten Abschnitt 23 auf, dessen Außendurchmesser so gewählt ist, daß das Druckelement 20 in der Bohrung 9 des Aufnahmeteiles 5 eine Gleitbewegung in axialer Richtung ausführen kann.

Ferner weist das Druckelement 20 an den ersten Abschnitt 23 und an das zweite Ende 22 angrenzend einen zweiten Abschnitt 24 mit einer gegen das zweite Ende 22 hin konisch zulaufenden Außenfläche auf, wobei der Kegelwinkel dem des Abschnittes 16 der Bohrung 9 des Aufnahmeteiles 5 entspricht. An dem zweiten Ende 22 ist eine koaxial ausgerichtete und zu dem zweiten Ende 22 hin offene kugelsegmentförmige Ausnehmung 25 zur Aufnahme des Kopfes 3 vorgesehen. Die kugelsegmentförmige Ausnehmung 25 weist einen Radius auf, der im wesentlichen gleich dem Radius des Kopfes 3 ist. In axialer Richtung ist die Abmessung der Ausnehmung 25 größer als ihr Radius. In der Wandung des Druckelementes 20 sind vier in Umfangsrichtung äquidistante achsenparallel ausgerichtete und zum zweiten Ende 22 hin offene Schlitze vorgesehen, deren Länge in axialer Richtung größer als der Radius der Ausnehmung 25 ist.

Ferner weist das Druckelement 20 in seinem zylinderförmigen ersten Abschnitt 23 eine zur Zylinderachse koaxiale und in die Ausnehmung 25 mündende Bohrung 26 zum Hindurchführen eines mit der Ausnehmung 4 in Eingriff zu gelangenden Schraubwerkzeuges auf. An ihrem ersten Ende 21 weist die Oberfläche des Druckelementes 20 eine sich senkrecht zur Zylinderachse des ersten Abschnittes 23 erstreckende zylinderabschnittförmige Ausnehmung 27 auf. Der Krümmungsmittelpunkt liegt auf der Symmetrieachse. Die Tiefe der Ausnehmung ist maximal gleich dem Radius der Ausnehmung. Der Krümmungsradius der Ausnehmung 27 entspricht dem Krümmungsradius des Grundes der U-förmigen Ausnehmung 10 des Aufnahmeteiles 5. Die Längsschlitze 28 dienen dazu, daß der den Kopf 3 umfassende konische Abschnitt 24 des Druckelementes so aufgebogen werden kann, daß das Druckelement 20 mit seiner kugelsegmentförmigen Ausnehmung 25 über den Kopf 3 der Schraube geschoben werden kann.

Das Druckelement 20 weist auf seinem Mantel zwei einander gegenüberliegend angeordnete und sich senkrecht zur Symmetrieachse erstreckende Senkbohrungen 29 auf. Entsprechende Kröpfbohrungen 17 sind im Mantel des Aufnahmeteiles 5 vorgesehen.

Es ist ferner eine die beiden Seitenschenkel 11, 12 von außen umfassendes Element in Form einer Überwurfmutter 40 zum Fixieren des aufzunehmenden Stabes 15 vorgesehen mit einem dem Außengewinde 14 der beiden Schenkel des Aufnahmeteiles 5 entsprechenden Innengewinde. Weiter ist ein als Gewindeschraube ausgebildetes Fixierelement 45 vorgesehen. Das Fixierelement 45 weist ein mit dem Innengewinde 13 der Schenkel 11, 12 zusammenwirkendes Außengewinde 46 auf zum Einschrauben in die U-förmige Ausnehmung 10 des Aufnahmeteiles 5. Ferner weist das Fixierelement 45 eine sechskantförmige Ausnehmung 46 zum Eingreifen mit einem Schraubwerkzeug auf.

Alle Teile des beschriebenen Verankerungselementes sind aus einem körperfreundlichen Material, insbesondere aus Titan gefertigt.

Zur Vorbereitung wird zuerst das Druckelement 20 so über den Kopf 3 geschoben, daß dieser von dem Rand der kugelsegmentförmigen Ausnehmung 25 umfaßt wird. Durch die bei dem Aufsetzen entstehenden Kräfte wird dabei das Druckelement an den Längsschlitzen 28 etwas auseinandergebogen, so daß der Schraubenkopf 3 in die kugelsegmentförmige Ausnehmung 25 eingeführt werden kann bzw. das Druckelement auf den Kopf 3 aufschnappt. Durch anschließendes leichtes Zusammendrücken kann eine formschlüssige Verbindung zwischen dem Rand der kugelsegmentförmigen Ausnehmung 25 und dem Schraubenkopf 3 erreicht werden.

Dann wird der Gewindeschaft 2 von dem ersten Ende 6 in das Aufnahmeteil eingeführt, bis die kegelige Außenfläche des zweiten Abschnittes 24 des Druckelementes 20 in Reibkontakt mit der Wand des konischen Abschnittes 16 der Bohrung 9 gelangt. Das Druckelement 20 wird dabei so gedreht, daß die Achse der zylinderabschnittförmigen Ausnehmung 27 genau in der Symmetrieebene des U-förmigen Kanales 10 des Aufnahmeteiles 5 liegt. Diese Position wird durch ein leichtes Ankröpfen über die Kröpfbohrungen 17, 29 arretiert, ohne daß dadurch eine ausreichende Bewegung des Druckelementes 20 in Richtung der Symmetrieachse 8 des Aufnahmeteiles behindert würde.

Im Betrieb wird nach dem Einschrauben des Schraubelementes 1 der Stab 15 in das Aufnahmeteil 5 über die U-förmige Ausnehmung 10 von außen eingelegt. Nach der Einstellung der gewünschten Relativstellung des Verankerungselementes zu dem Stab 15 wird der Stab 15 durch Festschrauben der Überwurfmutter 40 festgeklemmt. Dieses Festziehen der Überwurfmutter 40 bewirkt, wie insbesondere aus Fig. 1 ersichtlich ist, daß über den Stab 15 auf das Druckelement 20 eine Kraft F in Richtung der Symmetrieachse 8 ausgeübt wird, so daß das Druckelement 20 mitsamt Schraubenkopf 3 so in das Aufnahmeteil gedrückt wird, daß der konische zweite Abschnitt 24 des Druckelementes 20 vollständig in den konischen Abschnitt 16 der Bohrung 9 des Aufnameteiles geschoben wird.

Wenn die konischen Flächen des zweiten Abschnittes 24 des Druckelementes vollständig an den Flächen der Bohrung 9 des Aufnahmeteils anliegen, wird, wie insbesondere in Fig. 1 gezeigt ist, durch das Aufnahmeteil eine Kraft Fᵣₑₛ in zur Symmetrieachse 8 radialer Richtung auf das Druckelement ausgeübt, durch die eine Bewegung des Schraubenkopfes in der Ausnehmung 25 auch dann blockiert wird, wenn zum Zwecke einer Nachjustierung der Relativstellung von Stab 15 und Aufnahmeteil 5 die Überwurfmutter 40 gelöst wird, so daß die beim vorhergehenden Arretieren des Stabes fixierte Winkelstellung von Schraubenelement 1 und Aufnahmeteil 5 fixiert bleibt.

Da der Kegelwinkel so gewählt ist, daß Selbsthemmung der so gebildeten Verbindung zwischen der konischen Fläche des Abschnittes 16 der Bohrung und des Abschnittes 24 des Druckelementes auftritt, kann sich diese Verbindung nicht selbständig lösen. Zum Lösen ist vielmehr eine zusätzliche äußere Kraft von erheblicher Größe nötig, um die der Lösung entgegenwirkende Reibungskraft zu überwinden.

Im Betrieb ist in mehreren benachbarten Wirbelsäulensegmenten jeweils ein entsprechendes Verankerungselement angeordnet, wobei Segment für Segment eine Feineinstellung, bei der die Angriffsposition der einzelnen Verankerungselemente an dem Stab relativ zur Längsachse des Stabes einzustellen ist, durchgeführt wird. Da das Druckelement bei der Justierung der Stellung des Schraubenkopfes in dem Aufnahmeteil beim erstmaligen Einstellen der Lage des Aufnahmeteiles relativ zu dem Stab fest in das Aufnahmeteil 5 hineingedrückt wird und somit die Stellung des Schraubenkopfes 3 blockiert, wobei diese Blockierung aufgrund der Selbsthemmung im normalen Betrieb nicht mehr gelöst werden kann, kann zur Feineinstellung der Position des Aufnahmeteiles relativ zum Stab die Überwurfmutter 40 wieder gelöst werden.

Wenn die durch die Feineinstellung erhaltene Position des Aufnahmeteiles 5 relativ zu dem Stab 15 bestimmt ist, wird die Überwurfmutter wieder fest angezogen. Anschließend wird das Fixierelement 45 zwischen die Schenkel 11, 12 in Richtung des Stabes zum Arretieren der Überwurfmutter eingeschraubt.

Der oben beschriebenen Kegelwinkel liegt vorzugsweise in einem Bereich zwischen 2° und 10°. Besonders bevorzugt liegt der Winkel in einem Bereich von 2° bis 5°.

## Patentansprüche

1. Verankerungselement mit
einer Knochenschraube, die ein einen Gewindeabschnitt (2) und einen kugelsegmentförmigen Abschnitt besitzenden Kopf (3) aufweisendes Schraubenelement (1) und ein Aufnahmeteil (5) für die Aufnahme des Kopfes (3) des Schraubenelementes und eines mit dem Verankerungselement zu verbindenden Stabes (15) aufweist,
wobei das Aufnahmeteil (5) ein erstes Ende (6) und ein diesem gegenüberliegendes zweites Ende (7), eine das erste und-das zweite Ende schneidende Symmetrieachse (8), eine zu der Symmetrieachse koaxiale Bohrung (9) zum Hindurchführen des Gewindeabschnittes (2) und einen in einem an das erste Ende (6) angrenzenden ersten Bereich mit im wesentlichen U-förmigen Querschnitt mit zwei freien, ein Innengewinde (13) aufweisenden Schenkeln (11, 12) zur Aufnahme des einzusetzenden Stabes (15) aufweist,
dadurch gekennzeichnet, daß
die Bohrung (9) in einem an das zweite Ende (7) angrenzenden zweiten Bereich (16) gegen das zweite Ende (7) hin mit einem Kegelwinkel konisch verjüngt ist und daß ein den Schraubenkopf (3) von seiner dem Gewindeabschnitt (2) abgewandten Seite her umfassendes Druckelement (20) vorgesehen ist, dessen Außenfläche in einem den Schraubenkopf (3) seitlich umfassenden Bereich (24) gegen das zweite Ende (7) hin kegelig ausgebildet ist, wobei der Kegelwinkel dem des zweiten Bereiches (16) entspricht und so gewählt ist, daß bei vollständig in die Bohrung (9) eingeschobenem Druckelement (20) zwischen dessen kegeligem Bereich (24) und dem konischen zweiten Bereich (16) der Bohrung (9) Selbsthemmung auftritt.

2. Verankerungselement nach Anspruch 1, dadurch gekennzeichnet, daß der Kegelwinkel in einem Bereich von etwa 2° bis annähernd 10° liegt.

3. Verankerungselement nach Anspruch 1 oder 2, dadurch gekennzeichnet, daß der Kegelwinkel etwa 4° beträgt.

4. Verankerungselement nach einem der Ansprüche 1 bis 3, dadurch gekennzeichnet, daß das Druckelement (20) ein erstes Ende (21) und ein diesem gegenüberliegendes zweites Ende (22) und einen an das erste Ende (21) angrenzenden im wesentlichen zylinderförmigen ersten Abschnitt (23), dessen Außendurchmesser so gewählt ist, daß das Druckelement in der Bohrung (9) gleiten kann, und angrenzend an den ersten Abschnitt (23) einen kegelstumpfförmigen zweiten Abschnitt (24), dessen Außenfläche gegen das zweite Ende (22) hin kegelig zuläuft, aufweist.

5. Verankerungselement nach Anspruch 4, dadurch gekennzeichnet, daß das Druckelement (20) in seinem zweiten Bereich (24) eine zu dem zweiten Ende hin offene kugelsegmentförmige Ausnehmung (25) zur Aufnahme des Schraubenkopfes (3) aufweist.

6. Verankerungselement nach Anspruch 5, dadurch gekennzeichnet, daß das Druckelement (20) eine zur Zylinderachse des ersten Abschnittes (23) koaxiale und in die kugelsegmentförmige Ausnehmung (25) mündende Bohrung zum Hindurchführen eines mit dem Schraubenkopf (3) in Eingriff zu gelangenden Schraubwerkzeuges aufweist.

7. Verankerungselement nach einem der Ansprüche 1 bis 6, dadurch gekennzeichnet, daß das Druckelement (20) auf seiner dem Schraubenkopf (3) abgewandten Seite eine zylinderabschnittförmige Ausnehmung (27) aufweist, deren Zylinderachse senkrecht zu der Symmetrieachse (8) verläuft und deren Krümmungsradius dem Krümmungsradius des Grundes der durch die beiden Schenkel (11, 12) begrenzten U-förmigen Ausnehmung (10) des Aufnahmeteiles entspricht.

8. Verankerungselement nach einem der Ansprüche 5 bis 7, dadurch gekennzeichnet, daß das Druckelement (20) eine Mehrzahl von Längsschlitzen (28), deren Längsachse parallel zu der Zylinderachse des ersten Abschnittes (23) verläuft und die zu dem zweiten Ende (22) hin offen sind, aufweist.

9. Verankerungselement nach Anspruch 8, dadurch gekennzeichnet, daß zwei einander gegenüberliegende Längsschlitze (28) vorgesehen sind, deren Längsachse in einer Ebene liegt, welche durch die Zylinderachse des ersten zylinderförmigen Abschnittes (23) hindurch geht und sich senkrecht zu der Zylinderachse der Ausnehmung (27) erstreckt, wobei die Längsschlitze sich nahezu bis an das erste Ende (21) des Druckelementes erstrecken.

10. Verankerungselement nach einem der Ansprüche 1 bis 9, dadurch gekennzeichnet, daß das Druckelement (20) mit dem eingesetzten Schraubenkopf (3) durch Kröpfen in dem Aufnahmeteil (5) gegen Verdrehung gesichert ist.

11. Verankerungselement nach einem der Ansprüche 5 bis 10, dadurch gekennzeichnet, daß die Tiefe der Ausnehmung (25) in axialer Richtung gesehen größer als ihr Radius ist.

12. Verankerungselement nach einem der Ansprüche 1 bis 11, dadurch gekennzeichnet, daß ein die beiden freien Schenkel (11, 12) von außen umfassendes Element in Form einer Überwurfmutter (40) vorgesehen ist, deren Innengewinde mit dem Außengewinde (14) im Bereich der Schenkel zusammenwirkt, und zum Fixieren der Überwurfmutter (40) ein ein Außengewinde aufweisendes Fixierelement (45) vorgesehen ist, welches mit dem Innengewinde (13) der Schenkel (11, 12) in Eingriff gelangt.

## Claims

1. Anchor element having a bone screw which has a screw element (1), having a threaded section (2) and a head (3) with spherical-segment-shaped section, and a receiving part (5) for receiving the head (3) of the screw element and of a rod (15) which is to be connected to the anchor element, the receiving part (5) having a first end (6) and a second end (7), located opposite the first, an axis of symmetry (8), which intersects the first and the second ends, a bore (9), which is coaxial with the axis of symmetry and intended for the through-passage of the threaded section (2), and a in a first region, which adjoins the first end (6) and has an essentially U-shaped cross-section with two free legs (11, 12) which have an internal thread (13) and are intended for receiving the rod (15) which is to be inserted, characterized in that in a second region (16), which adjoins the second end (7), the bore (9) is tapered conically at a cone angle in the direction of the second end (7), and in that there is provided a pressure-exerting element (20) which encloses the screw head (3) from its side remote from the threaded section (2) and of which the outer surface is designed conically in the direction of the second end (7) in a region (24), which encloses the screw head (3) laterally, the cone angle corresponding to that of the second region (16) and being selected such that, when the pressure-exerting element (20) has been pushed into the bore (9) to the full extent, self-locking takes place between its conical region (24) and the conical, second region (16) of the bore (9).

2. Anchor element according to Claim 1, characterized in that the cone angle is in a range of from approximately 2° to more or less 10°.

3. Anchor element according to Claim 1 or 2, characterized in that the cone angle is approximately 4°.

4. Anchor element according to one of Claims 1 to 3, characterized in that the pressure-exerting element (20) has a first end (21) and a second end (22), located opposite the first, and an essentially cylindrical, first section (23), which adjoins the first end (21) and of which the external diameter is selected such that the pressure-exerting element can slide in the bore (9), and, adjoining the first section (23), a frustoconical, second section (24), of which the outer surface tapers conically in the direction of the second end (22).

5. Anchor element according to Claim 4, characterized in that, in its second region (24), the pressure-exerting element (20) has a spherical-segment-shaped recess (25) which is open in the direction of the second end and is intended for receiving the screw head (3).

6. Anchor element according to Claim 5, characterized in that the pressure-exerting element (20) has a bore which is coaxial with the cylinder axis of the first section (23), opens out into the spherical-segment-shaped recess (25) and is intended for the through-passage of a screwing-in tool which can engage with the screw head (3).

7. Anchor element according to one of Claims 1 to 6, characterized in that, on its side remote from the screw head (3), the pressure-exerting element (20) has a recess (27) which is in the form of a cylinder section, of which the cylinder axis runs perpendicularly to the axis of symmetry (8) and of which the radius of curvature corresponds to the radius of curvature of the base of the U-shaped recess (10) of the receiving part, said recess being bounded by the two legs (11, 12).

8. Anchor element according to one of Claims 5 to 7, characterized in that the pressure-exerting element (20) has a plurality of longitudinal slits (28), of which the longitudinal axis runs parallel to the cylinder axis of the first section (23) and which are open in the direction of the second end (22).

9. Anchor element according to Claim 8, characterized in that there are provided two mutually opposite longitudinal slits (28), of which the longitudinal axis is located in a plane which passes through the cylinder axis of the first, cylindrical section (23) and extends perpendicularly to the cylinder axis of the recess (27), the longitudinal slits extending virtually as far as the first end (21) of the pressure-exerting element.

10. Anchor element according to one of Claims 1 to 9, characterized in that the pressure-exerting element (20) with the screw head (3) inserted therein is secured against rotation by angling in the receiving part (5).

11. Anchor element according to one of Claims 1 to 10, characterized in that, as seen in the axial direction, the depth of the recess (25) is greater than the radius thereof.

12. Anchor element according to one of Claims 1 to 11, characterized in that there is provided an element which encloses the two free legs (11, 12) from the outside and is in the form of a union nut (40), of which the internal thread interacts with the external thread (14) in the region of the legs, and provided for fixing the union nut (40) is a fixing element (45) which has an external thread and engages with the internal thread (13) of the legs (11, 12).

## Revendications

1. Elément d'ancrage avec une vis à os, qui présente un élément de vis (1) présentant une partie filetée (2) et une partie en forme de segment sphérique et une pièce réceptrice (5) pour le logement de la tête (3) de l'élément de vis et d'une barre (15) à relier avec l'élément d'ancrage, la pièce réceptrice (5) présentant une première extrémité (6) et une deuxième extrémité (7) opposée à celle-ci, un axe de symétrie (8) coupant la première et la deuxième extrémité, un perçage (9) situé sur le même axe que l'axe de symétrie pour le passage de la partie filetée (2) et une première zone contiguë à la première extrémité (6) avec une section sensiblement en forme de U avec deux branches (11, 12) libres et présentant un filetage intérieur (13) pour le logement de la barre à insérer (15), caractérisé en ce que le perçage (9) est rétréci en forme de cône dans une deuxième zone (16) contiguë à la deuxième extrémité (7) en direction de la deuxième extrémité (7) avec un angle de cône et en ce qu'il est prévu un élément de pression (20) enveloppant la tête de vis (3) par son côté opposé à la partie filetée (2), élément dont la surface extérieure est conçue en forme de cône dans une zone (24) enveloppant la tête de vis (3) par le côté en direction de la deuxième extrémité (7), l'angle de cône correspondant à celui de la deuxième zone (16) et étant choisi de telle façon que, lorsque l'élément de pression (20) est complètement enfoncé dans le perçage (9), il apparaisse un autoblocage entre la zone conique (24) et la deuxième zone (16) conique du perçage (9).

2. Elément d'ancrage selon la revendication 1, caractérisé en ce que l'angle de cône se situe dans une plage allant d'environ 2° à approximativement 10°.

3. Elément d'ancrage selon la revendication 1 ou 2, caractérisé en ce que l'angle de cône est d'environ 4°.

4. Elément d'ancrage selon l'une quelconque des revendications 1 à 3, caractérisé en ce que l'élément de pression (20) présente une première extrémité (21) et une deuxième extrémité (22) opposée à la première et une première partie (23) contiguë à la première extrémité (21) et sensiblement en forme de cylindre, dont le diamètre extérieur est choisi de telle façon que l'élément de pression peut glisser dans le perçage (9), et de façon contiguë à la première partie (23) une deuxième partie (24) en forme de cône tronqué, dont la surface extérieure va en direction de la deuxième extrémité (22) avec la forme d'un cône.

5. Elément d'ancrage selon la revendication 4, caractérisé en ce que l'élément de pression (20) présente dans sa deuxième zone (24) un évidement (25) en forme de segment sphérique et ouvert en direction de la deuxième extrémité pour le logement de la tête de vis (3).

6. Elément d'ancrage selon la revendication 5, caractérisé en ce que l'élément de pression (20) présente un perçage situé sur le même axe que l'axe de cylindre de la première partie (23) et débouchant dans l'évidement (25) en forme de segment sphérique pour le passage d'un outil de vissage en prise avec la tête de vis (3).

7. Elément d'ancrage selon l'une quelconque des revendications 1 à 6, caractérisé en ce que l'élément de pression (20) présente sur son côté opposé à la tête de vis (3) un évidement (27) en forme de partie cylindrique, dont l'axe de cylindre est perpendiculaire à l'axe de symétrie (8) et dont le rayon de courbure correspond au rayon de courbure du fond de l'évidement (10), en forme de U et limité par les deux branches (11, 12), de la pièce réceptrice.

8. Elément d'ancrage selon l'une quelconque des revendications 5 à 7, caractérisé en ce que l'élément de pression (20) présente un grand nombre de fentes longitudinales (28), dont l'axe longitudinal est parallèle à l'axe de cylindre de la première partie (23) et est ouvert en direction de la deuxième extrémité (22).

9. Elément d'ancrage selon la revendication 8, caractérisé en ce qu'il est prévu deux fentes longitudinales (28) opposées entre elles, dont l'axe longitudinal se situe dans un plan qui passe par l'axe de cylindre de la première partie (23) en forme de cylindre et s'étend perpendiculaire à l'axe de cylindre de l'évidement (27), les fentes longitudinales s'étendant pratiquement jusqu'à la première extrémité (21) de l'élément de pression.

10. Elément d'ancrage selon l'une quelconque des revendications 1 à 9, caractérisé en ce que l'élément de pression (20) avec la tête de vis (3) insérée est protégé contre la torsion grâce à un coude dans la pièce réceptrice.

11. Elément d'ancrage selon l'une quelconque des revendications 5 à 10, caractérisé en ce que la profondeur de l'évidement (25) est supérieure à son rayon vu dans le sens axial.

12. Elément d'ancrage selon l'une quelconque des revendications 1 à 11, caractérisé en ce qu'il est prévu un élément enveloppant les deux branches (11, 12) libres par l'extérieur sous la forme d'un écrou raccord (40), dont le filetage intérieur agit conjointement avec le filetage extérieur (14) dans la zone des branches, et un élément de fixation (45) présentant un filetage extérieur pour la fixation de l'écrou raccord (40), lequel est en prise avec le filetage intérieur (13) des branches (11, 12).
